(19) Europäisches Patentamt
European Patent Office
Office européen des brevets

(11) Publication number: 0 082 384 A1

(12) **EUROPEAN PATENT APPLICATION**

(21) Application number: 82111232.3

(22) Date of filing: 04.12.82

(51) Int. Cl.$^3$: C 07 C 147/10

(30) Priority: 17.12.81 US 331701

(43) Date of publication of application: 29.06.83 Bulletin 83/26

(84) Designated Contracting States: DE FR GB IT

(71) Applicant: Mobay Chemical Corporation
Penn Lincoln Parkway West
Pittsburgh, Pennsylvania 15205(US)

(72) Inventor: Sanderson, John R.
5306 Rambling Range
Austin Texas 78759(US)

(72) Inventor: Krishnan, Sivaram
159 Fairview Drive New Martinsville
West Virginia W. VA 26155(US)

(72) Inventor: Baron, Arthur L.
211 East Thistle Court
New Martinsville West Virginia 26155(US)

(74) Representative: Gremm, Joachim, Dr. et al,
Bayer AG c/o Zentralbereich Patente, Marken und Lizenzen
D-5090 Leverkusen 1, Bayerwerk(DE)

(54) **A process for the preparation of bis(4-hydroxyphenyl)biphenyl disulfone.**

(57) A process for the production of bis(4-hydroxyphenyl) biphenyl disulfone comprising reacting 4-4'-biphenyldisulfonyl chloride with monochlorobenzene in the presence of ferric chloride, dimethylsulfoxide and aqueous sodium hydroxide.

EP 0 082 384 A1

Croydon Printing Company Ltd

Mo-2314
PC-030

# A PROCESS FOR THE PREPARATION OF BIS(4-HYDROXYPHENYL)BIPHENYL DISULFONE

## BACKGROUND OF THE INVENTION

Bis(4-hydroxyphenyl)biphenyl disulfone has been found to be a useful monomer in preparing polymers such as polyurethanes, polycarbonates, polyethers, polyesters and polysulfones. Polycarbonates prepared using such a monomer are disclosed in U.S. Patent 3,269,986. Such polymers are generally useful for the preparation of films or fibers and for injection molded or extruded parts, blow molded articles and coatings.

Huismann, in U.S. Patent 2,288,282, discloses (see Example 8 of that patent) an alternative method for the preparation of the disulfone of the present invention. There, a hydrolysis of the intermediate diphenyl-4,4'-bis(4"-chlorophenylsulphone) is carried out in a methyl alcohol/hydroxide solution and results in a mixture comprising a relatively low amount of the bis-4-hydroxyphenyl-diphenyl disulfone.

The object of the present invention is to provide a novel process for the preparation of bis(4-hydroxyphenyl)biphenyl disulfone.

## SUMMARY OF THE INVENTION

The present invention pertains to a process for the preparation of bis(4-hydroxyphenyl)biphenyl disulfone comprising reacting 4,4'-biphenyldisulfonyl-chloride with monochlorobenzene in the presence of ferric chloride, dimethylsulfoxide and aqueous sodium hydroxide.

## DETAILED DESCRIPTION OF THE INVENTION

The process of the present invention may be represented by the following general reaction scheme:

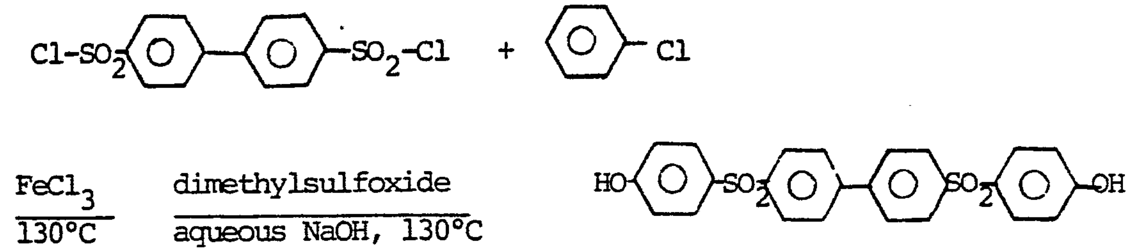


In one embodiment of the present invention, the process is carried out in two basic steps and may be represented by the following general reaction scheme:

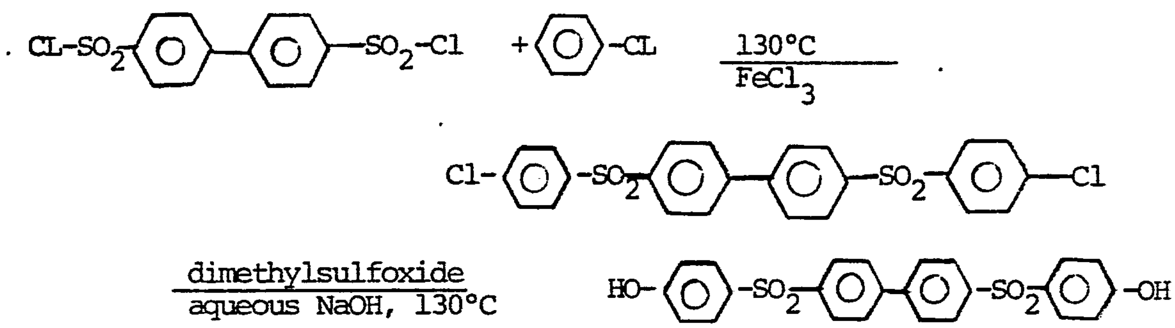


Upon the completion of the synthesis, the bis(4-hydroxyphenyl)biphenyl disulfone is recovered as a precipitate and is then separated, washed, dried and optionally then recrystallized.

The process of the invention generally produces high quality bis(4-hydroxyphenyl)biphenyl disulfone in yields of from about 40 to 50%, based on the theoretical yield.

The invention will be further illustrated, but is not intended to be limited, by the following examples.

## EXAMPLES

**EXAMPLE 1** (One Flask Procedure)

A three-necked 12 liter flask was equipped with a stirrer, condenser, heating mantle, thermometer

and a nitrogen purge. The outlet was connected to a bubbler (water or NaOH solution) to absorb the HCl evolved. The flask was purged with nitrogen. 4,4'-Biphenyldisulfonylchloride (1756 g, 5 moles), monochlorobenzene (3 liters, 29.6 moles) and anhydrous ferric chloride (81.0 g, 0.5 mole) were charged to the flask. The mixture was stirred, warmed to 130°C (±2°C) and maintained at this temperature for 15-20 hours. Dimethylsulfoxide (2 liters) was added, the pot temperature raised to 170°C and the monochlorobenzene distilled ( 2 liters). An additinal 2 liters of dimethylsulfoxide and 50% sodium hydroxide (4,000 g, 50 moles) were then added. The mixture was refluxed for at least 20 hours. The contents of the flask were poured into 10 liters of water and 100 g of celite added. This mixture was stirred and allowed to settle. Most of the liquid was decanted from the solids, and the remaining slurry filtered through a 1/4" pad of celite. The liquid was added dropwise to a vigorously stirred solution of 10% HCl. The solid was collected with suction, washed with 5 liters of 20% acetone in water, 5 liters of water and air dried to give a 93% yield of crude bis(4-hydroxyphenyl)biphenyl disulfone. The identity of the material was confirmed by its IR spectrum.

The material was purified in the following manner: Crude bis(4-hydroxyphenyl)biphenyl disulfone ( 200 g) was treated with 500 ml toluene and 400 ml ethyl acetate. This mixture was heated to 100-110°C, and the upper layer was decanted from the lower darker layer and was filtered. The solvent was removed and the residue recrystallized from acetic acid (50%

recovery). The off-white to grey solid was analyzed to be 95% bis(4-hydroxyphenyl)biphenyl disulfone by thin layer chromotography.

EXAMPLE 2 (Two Step Procedure)

A three-necked, 12 liter flask was equipped with a stirrer, condenser, heating mantle, thermometer, and a nitrogen purge. The outlet was connected to a bubbler (water or NaOH solution) to absorb the HCl evolved. The flask was purged with nitrogen, and monochlorobenzene (7.8 liters, 77 moles) and 4,4'-biphenyldisulfonylchloride (2.27 kg, 6.5 moles), was charged to the flask. The mixture was heated to 70°C and anhydrous ferric chloride (65 g, 0.4 mole) added. The reaction mixture was then heated to 130°C and maintained at 130 ± 2°C for 20 hours. The reaction mixture was then cooled to room temperature, and the product collected with suction. (The crude yield was 84%). This material was crystallized from dimethyl formamide (80% recovery) to yield white crystals (M.P. 269-272°C) of bis(4-chlorophenyl)biphenyl disulfone.

To a 12 liter three-necked flask fitted with a stirrer, a thermometer, a condenser, an addition funnel and a gas inlet and outlet, was added 10 liters of dimethyl sulfoxide (DMSO) and 1 kg (1.99 moles of bis(4-chlorophenyl)biphenyl disulfone. A slow stream of nitrogen was passed through the apparatus. The reaction mixture was heated to 70°C until the bis(4-chlorophenyl)biphenyl disulfone was completely in solution (1 hour). 50% Sodium hydroxide (1.6 kg) was added dropwise in 1 hour, and the color of the reaction mixture changed. After the addition of the sodium hydroxide was complete, the temperature of the reaction

mixture was raised to 130-140°C and held at this temperature for 18 hours. After cooling to room temperature, the reaction mixture was diluted with 30 liters of distilled water and was acidified to pH = 6. The precipitate thus separated was filtered, washed and dried at 130°C under vacuum. The crude product (752 g; 81% yield) was recrystallized from glacial acetic acid (50% recovery) as white powdery bis(4-hydroxyphenyl) biphenyl disulfone (M.P. 242-243°C).

COMPARISON EXAMPLE

The procedure described in U.S. Patent 2,288,282 -Example 8 - was followed as described below:

A mixture of a solution of 44.8 g of potassium hydroxide in about 6 ml of water and 100 g of methanol and 50.3 g of bis(4-chlorophenyl sulfonyl)biphenyl in an autoclave was heated at 150 to 155°C for 12 hours. The reaction mixture was allowed to cool whereupon the potassium salts separated were removed by filtration. The filtrate was evaporated. The evaporated residue was taken in water. This residue was not soluble in water indicating the formation partly of compound which is not a phenolic potassium salt. On pouring the diluted reaction mixture into an excess of dilute hydrochloric acid, a product (22.8 g) with melting point 114-121° was obtained.

NMR spectra of the product indicates significant formation of methoxy analogs (methoxy or dimethoxy) of the biphenyl of interest.

WHAT IS CLAIMED IS:

1. A process for the preparation of bis(4-hydroxyphenyl)biphenyl disulfone comprising reacting 4,4'-biphenyl-disulfonylchloride with monochlorobenzene in the presence of ferric chloride, dimethylsulfoxide and aqueous sodium hydroxide.

2. The process of Claim 1 wherein the 4,4'-biphenyldisulfonyl chloride is reacted with monochlorobenzene in the presence of ferric chloride to produce 4,4'-bis(4-chlorophenyl)-biphenyl disulfone, and the 4,4'-bis(4-chlorophenyl)biphenyl disulfone is subsequently heated in the presence of dimethyl sulfoxide and aqueous sodium hydroxide to produce said bis(4-hydroxyphenyl)biphenyl disulfone.

European Patent Office

# EUROPEAN SEARCH REPORT

0082384

Application number

EP 82 11 1232

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 C 147/10 |
| Y | EP-A-0 010 595 (MOBAY CHEM. CORP.)<br>* page 7, process 1 *<br>--- | 1,2 | |
| Y | BULLETIN OF THE CHEMICAL SOCIETY OF JAPAN, vol. 40, no. 7, July 1967<br>S. OAE et al.: "The alkaline hydrolyses of chlorophenyl phenyl sulfoxides and sulfones", pages 1716-1719. * the whole article *<br>----- | 1,2 | |
| | | | TECHNICAL FIELDS SEARCHED (Int. Cl. 3) |
| | | | C 07 C 147/00 |

The present search report has been drawn up for all claims

| Place of search | Date of completion of the search | Examiner |
|---|---|---|
| THE HAGUE | 12-01-1983 | GRAMAGLIA R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82